# EUROPEAN PATENT APPLICATION

(11) **EP 4 609 783 A1**
(43) Date of publication of application: **03.09.2025**
(21) Application number: 25158461.1
(22) Date of filing: 18.02.2025
(51) Int. Cl.: A61B 5/00

(54) **PENIS MEASUREMENT SYSTEM AND METHOD**

(30) Priority: 01.03.2024 TW 113107578
(71) Applicant: Tung, Sung-Chen, New Taipei City (TW)
(72) Inventor: Tung, Sung-Chen, New Taipei City (TW)
(74) Representative: Tiburzi, Andrea

(57) **Abstract**

A penis measurement system includes a user device (200) and a penis measurement device (100). An external soft casing (120) encases a main shell (110) of the penis measurement device (100). When at least one strap of the external soft casing (120) is strapped on a penis (300), the external soft casing (120) fixes the main shell (110) on the penis (300), allowing a detection airbag (130) protruding from the main shell (110) to down-press the penis (300). The detection airbag (130) deforms and presses a sensor chip (20) in the main shell (110). A processing unit (10) receives a pressure sensor signal from the sensor chip (20) and a gyroscope signal from a gyroscope (50) in the main shell (110), and outputs a hardness value and a gyroscope value to a user device (200), quantifying a hardness of the penis (300).

## Description

### 1. Field of the Invention

The present invention relates to a penis measurement system and method, capable of more accurately detecting and recording male physiological data during sexual activities.

### 2. Description of the Related Art

For the purpose of avoiding any misunderstandings, the present application refers to males and females as traditionally defined biological males and biological females.

In the technical field of gender physiological studies, a hardness of penis determines whether a sexual intercourse with a female can be successful. The hardness of penis, however, currently cannot be unbiasedly, scientifically, quantitatively gauged, but only qualitatively rated. For this reason, professionals in the said technical field need an unbiased apparatus to obtain the hardness of penis for the purpose of scientifically evaluating the successfulness of the penis to have sexual intercourse.

In the technical field of gauging a hardness of a piece of rubber, traditionally the rubber is down-pressed with a force, and then upon release, a force of the rubber bouncing back is gauged and measured. This hardness measurement of the piece of rubber is often conducted by a Shore durometer with a C-shaped protruding tip. The protruding tip of the Shore durometer is internally connected to a fixed spring, thus by down-pressing the protruding tip into an object with a fixed depth and measuring how much the protruding tip is being pushed back by the object, the hardness of the object is gauged and measured. In other words, a height of the object pushing back the protruding tip is measured by the Shore durometer. More particularly, by manually or using machine to down-press the protruding tip 90 degrees into a surface of the object, the surface of the object would bounce back the protruding tip and exert pressure of the fixed spring. The distance of the object pushing back the protruding tip is positively correlated to how much force is exerted on the fixed spring. As a result, the harder the object is, the shallower the protruding tip is able to be pressed into the surface of the object, and the more distance the object pushes back the protruding tip, thus adding more pressure on the fixed spring of the Shore durometer and causing the Shore durometer to display a greater measurement value. Vice versa, the softer the object is, the deeper the protruding tip is able to be pressed into the surface of the object, and the less distance the object pushes back the protruding tip, thus putting lesser pressure on the fixed spring of the Shore durometer and causing the Shore durometer to display a lesser measurement value. In short, the greater the pressure exerted by the surface of the object bouncing back, the greater measurement value is measured by the Shore durometer, thus signifying the object having a greater measured hardness.

In the technical field of measuring the hardness of penis, currently the following problems remain to be solved.

Problem 1: when attempting to measure the hardness of penis, a detection of the male nocturnal penile tumescence (NPT) cannot be effectively conducted. According to known physiological studies, males tend to have NPT naturally during rapid-eye-movement (REM) sleeps, and the hardness of the penis as well as the counts of erections during NPT are viewed as standard unbiased data usable for evaluating a male physiological status. In a traditional practice, this hardness measurement is conducted by attaching a stamp paper to a root of the penis at night. In the next morning, if the stamp paper is torn by the penis, the penis is hastily concluded to have enough hardness, when enlarging for having an erection during NPT, to overcome a resistance force imposed by the stamp paper to the penis. However, this traditional practice only measures a degree of enlargement of the penis in a single instance, thus unable to provide statistical data for erection counts. The traditional practice also cannot accurately and quantitatively measure the degree of enlargement of the penis as well as the hardness of the penis.

A current alternative to measure the penis is by using Rigiscan^{®}. However, although Rigiscan^{®} theoretically is able to measure erection counts, in practice, a fixing contraption of the Rigiscan^{®} often slips away from the penis during a man's sleep. Furthermore, the Rigiscan^{®} is too large in size, thus when the man turns during sleep, the Rigiscan^{®} often prevents the man from sleeping comfortably, and as a result of bad sleep, the results measured by the Rigiscan^{®} may not most accurately represent the data gathered for an erection during NPT. Due to the above reasons, the Rigiscan^{®} still leaves desirable improvements for one to more effectively conduct measurements for the erection of the penis during NPT.

Problem 2: currently the hardness of a penis cannot be scientifically measured. A traditional clinical way of measuring the hardness of a penis often uses erectile hardness score (EHS). The EHS is first published in *The Journal of Sexual Medicine*, a famous medical journal, under "Validation of the Erection Hardness Score" as a research report by scholar Mulhall in 2007. Subsequently, Pfizer, a famous biopharmaceutical company, adopts the results published by Mulhall in the research report into four degrees of erectile hardness meters and created four corresponding penis models to qualitatively represent the four different degrees of erectile hardness. As a result, when a man self-describes the hardness of his penis in retrospect, the four penis models are provided to aid the man in qualitatively identifying the hardness of his penis. In other words, the man may self-identify his penis to have the same hardness of one of the four options. However, such a patient-reported outcome (PRO) is highly susceptible to being biased, and the biological sampling method is also not well-regulated. An individual may be hazy about his own penis hardness, resulting in extremely wide margin of error, especially when compared to other individual's own qualitative measurements. For the above reasons, currently the hardness of a penis cannot be scientifically measured, and the measurement of the hardness of a penis needs further improvements.

For reference, according to the research paper: Miranda, E., Hallan, B., Nascimento, B., Paul, G., Carneiro, F., De Bessa Junior, J., & Miranda, A. (2023). (121) "Validation of a Digital Rigidometer for the Evaluation of Erection Hardness During In-office Rigidity Assessment in Patients with Erectile Dysfunction," The Journal of Sexual Medicine, 20, when a penis cannot overcome a first degree (equivalent of 0.51 kilograms, or roughly about 5 Newtons) of down-pressing obstacle force or a second degree (equivalent of 0.75 kilograms, or roughly about 7.35 Newtons) of down-pressing obstacle force, the penis would bend and be unable to overcome the obstacle force imposed by a vagina, thus the penis would not be able to successfully have a sexual intercourse with the vagina. However, when the penis is able to overcome at least third degree (equivalent of 1.2 kilograms, or roughly about 11.76 Newtons) of down-pressing obstacle force, the penis would be able to remain straight and hard for overcoming the obstacle force imposed by the vagina and have a successful sexual intercourse. The down-pressing obstacle force is stress exerted by surfaces of the vagina towards a tip of the penis facing the vagina.

Problem 3: when using the Rigiscan^{®} to measure the penis, the Rigiscan^{®} is only able to measure up to roughly 60% of the hardness of the enlarged penis. According to the research paper: Allen, R. P., Smolev, J. K., Engel, R. M., & Brendler, C. B. (1993). "Comparison of RigiScan and formal nocturnal penile tumescence testing in the evaluation of erectile rigidity." The Journal of Urology, 149(5), 1265-1268, the Rigiscan^{®} apparatus includes a belt bonding a root part of the penis and a corona of glans of the penis. By investigating a change of tightness of the belt, the Rigiscan^{®} is used to measure the hardness of the penis. However, according to research responses from the research paper: Guay, A. T., & Heatley, G. J. (1994). "Re: Comparison of RigiScan and formal nocturnal penile tumescence testing in the evaluation of erectile rigidity." The Journal of Urology, 152(1), 171-171, a discovery is made by the researcher that minor abnormality of the erected penis cannot be accurately measured when the penis is enlarged to exceed 60% of the hardness measurement (measurement of the tightness of the belt) from the apparatus. For this reason, the Rigiscan^{®} cannot be considered as an accurate apparatus for measuring the hardness of the penis.

Problem 4: currently no apparatus is capable of measuring the hardness of the penis during a sexual intercourse. According to the research paper: Timm, G. (1999). "Axial penile buckling forces vs Rigiscan (TM) radial rigidity as a function of intracavernosal pressure: why Rigiscan does not predict functional erections in individual patients-Editorial comment." INTERNATIONAL JOURNAL OF IMPOTENCE RESEARCH, 11(6), 338-339, although an axial hardness of the penis is discussed by the article, no practical methodology is introduced for measuring the hardness of the penis during a sexual intercourse. For this reason, the research paper only presents theoretical discussions, and does not propose a practical solution of measuring the hardness of the penis during a sexual intercourse.

To overcome the aforementioned problems, the present invention provides a penis measurement system and method, capable of unbiasedly, scientifically, and quantitatively measuring a hardness data of the penis in a sexual intercourse and presenting a solution to the aforementioned problems.

The penis measurement system of the present invention includes:
a penis measurement device, configured to be fixed on a penis of a user, and including:
   a main shell, including a sensor opening;
   a detection airbag, positioned inside of the main shell, and protruding outwards from the sensor opening;
   a sensor chip, mounted inside of the main shell, and contacting the detection airbag;
   a communication unit, mounted inside of the main shell;
   a processing unit, mounted inside of the main shell, and electrically connected to the sensor chip and the communication unit;
   an external soft casing, encasing the main shell, and including at least one strap configured to be fixed on the penis;
   wherein when the at least one strap is strapped on the penis, the external soft casing fixes the main shell to be on top of a root part of a penis, the detection airbag contacts the penis through the external soft casing, and the detection airbag, from top of the root part of the penis, presses down onto the penis with a down-pressing pressure;
   wherein when the detection airbag receives a bounce-back pressure corresponding to the down-pressing pressure from the penis, the detection airbag deforms and presses onto the sensor chip, thus allowing the sensor chip to generate a pressure sensor signal corresponding to a stress exerted onto the sensor chip by the detection airbag; the sensor ship sends the pressure sensor signal to the processing unit, and the processing unit calculates and outputs a hardness value according to the pressure sensor signal;
a user device, communicatively connected to the communication unit of the penis measurement device; wherein the user device receives the hardness value outputted by the processing unit via the communication unit of the penis measurement device.

Since the external soft casing fixes the main shell to be on top of the root part of the penis when the at least one strap of the penis measurement device is strapped on the penis, when the user is having a sexual intercourse, the penis measurement device on the penis would not hinder the penis from moving in back and forth motions. Simultaneously, the detection airbag that is contacting the penis from top of the penis is able to receive and detect the bounce-back pressure corresponding to the down-pressing pressure.

To generalize, the harder the penis is, the less the penis would change its shape when receiving the down-pressing pressure, hence the penis would produce the greater bounce-back pressure corresponding to the down-pressing pressure. As the detection airbag receives the greater bounce-back pressure, the detection airbag would deform more to squeeze more onto the sensor chip, thus exerting more stress onto the sensor chip. As the sensor chip receives the greater stress, the sensor chip outputs the greater pressure sensor signal to the processing unit, thus allowing the processor unit to calculate the greater hardness value. In short, when the detection airbag is squeezed by the penis, the higher the detection airbag pressure is raised by the squeeze, the greater the detection airbag would deform and squeeze the sensor chip, hence representing having the greater hardness of the penis.

Vice versa, the softer the penis is, the greater the penis would change its shape when receiving the down-pressing pressure, hence the penis would produce the lesser bounce-back pressure corresponding to the down-pressing pressure. As the detection airbag receives the lesser bounce-back pressure, the detection airbag would deform less to squeeze less onto the sensor chip, thus putting lesser stress onto the sensor chip. As the sensor chip receives the lesser stress, the sensor chip outputs the lesser pressure sensor signal to the processing unit, thus allowing the processor unit to calculate the lesser hardness value.

As a result, the penis measurement device is able to overcome the aforementioned problems 1 to 4 for assisting the user to unbiasedly, scientifically, and quantitatively measure the hardness value of the penis when having a sexual intercourse. Furthermore, as the user device is able to receive the hardness value, more subsequent applications and calculations relating to the hardness value may be provided to the user through the user device.

The penis measurement device also includes a gyroscope. The gyroscope is configured for monitoring a degree of back and forth moving motion of the user's buttocks when the user is having a sexual intercourse. The gyroscope is mounted inside of the main shell, and the gyroscope is electrically connected to the processing unit. The gyroscope generates a gyroscope signal according to movement changes of the penis measurement device and sends the gyroscope signal to the processing unit. The processing unit calculates a gyroscope value according to the gyroscope signal, and the processing unit controls the communication unit to send the gyroscope value to the user device. In an embodiment, the user device is a smart phone.

A penis measurement method of the present invention is executed by the user device, and the penis measurement method includes the following steps:
recording a data receiving time for receiving a hardness value and a gyroscope value outputted from a penis measurement device;
uploading the hardness value, the gyroscope value, and the data receiving time to a cloud server;
continuously receiving the hardness value, the gyroscope value, and the data receiving time, and constructing a data changing graph according to the hardness value, the gyroscope value, and the data receiving time; and
displaying the data changing graph through a display.

As such, the data generated from the user having the sexual intercourse is able to be simultaneously uploaded and stored in the cloud server. The user may subsequently access the stored data in the cloud server. The user may also conveniently look at the data changing graph displayed on the display for gaining insights towards the data changes relating to the sexual intercourse over a period of time. This allows the user to unbiasedly analyze a history of the user's physiological changes during the period of time when the user is having the sexual intercourse.

### IN THE DRAWINGS

Fig. 1 is a block diagram of an embodiment of a penis measurement system of the present invention.
Fig. 2 is an exterior perspective view of a penis measurement device of the penis measurement system of the present invention.
Fig. 3 is an exploded perspective view of the penis measurement device of the penis measurement system of the present invention.
Fig. 4 is another exploded perspective view of the penis measurement device of the penis measurement system of the present invention.
Fig. 5 is a side-perspective view of the penis measurement device of the penis measurement system of the present invention.
Fig. 6 is a cross-sectional perspective view of the penis measurement device of the penis measurement system of the present invention.
Fig. 7 is a perspective view of using the penis measurement device of the penis measurement system of the present invention.
Fig. 8 is another perspective view of using the penis measurement device of the penis measurement system of the present invention.
Fig. 9 is a flow chart of a penis measurement method of the present invention.
Fig. 10 is another flow chart of a penis measurement method of the present invention.
Fig. 11 is another flow chart of a penis measurement method of the present invention.
Fig. 12 is a perspective view of a user device of the penis measurement system of the present invention displaying a first option page.
Fig. 13 is a perspective view of the user device of the penis measurement system of the present invention displaying a second option page.
Fig. 14 is a perspective view of a user device of the penis measurement system of the present invention displaying a calibration to zero page.
Fig. 15 is a perspective view of a user device of the penis measurement system of the present invention displaying a real-time data page.
Fig. 16 is a perspective view of a user device of the penis measurement system of the present invention displaying a data result page.
Fig. 17 is a perspective view of a user device of the penis measurement system of the present invention displaying a historical records page.

The present invention provides a penis measurement system and method, capable of assisting a user to unbiasedly, scientifically, and quantitatively measure a hardness of penis when having sexual activities, such as when having a sexual intercourse. As such, the present invention is able to resolve the following problems: a problem that patient-reported outcome (PRO) of self-assessed penis hardness is highly susceptible to being biased and cannot be scientifically adopted; a problem that when using the Rigiscan^{®} to measure the penis, the Rigiscan^{®} is only able to measure up to roughly 60% of the hardness of the enlarged penis; and a problem of currently having no capable apparatus for measuring the hardness of the penis during a sexual intercourse.

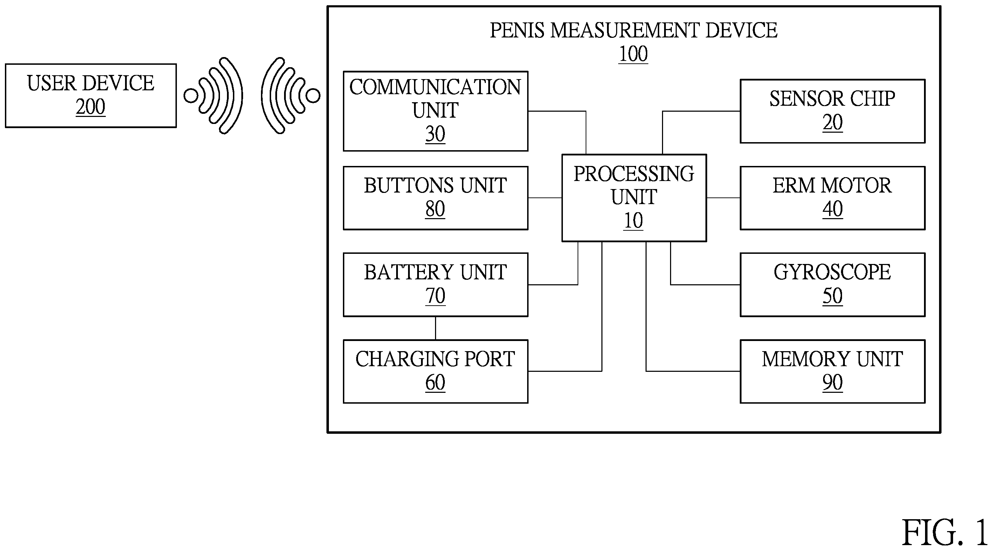

With reference to Fig. 1, the present invention provides a penis measurement system. The penis measurement system includes a penis measurement device 100 and a user device 200. The user device 200 is communicatively connected to the penis measurement device 100. The penis measurement device 100 is configured to be fixed on the user's penis, thus assisting to effectively measure physiological data related to the penis. Furthermore, regardless of what conditions the user is in, for example, regardless of the user is sleeping soundly and having nocturnal penile tumescence (NPT), or the user is fantasizing and masturbating, or the user is having a sexual intercourse with his partner, the present invention would be able to unbiasedly measure the physiological data related to the penis.

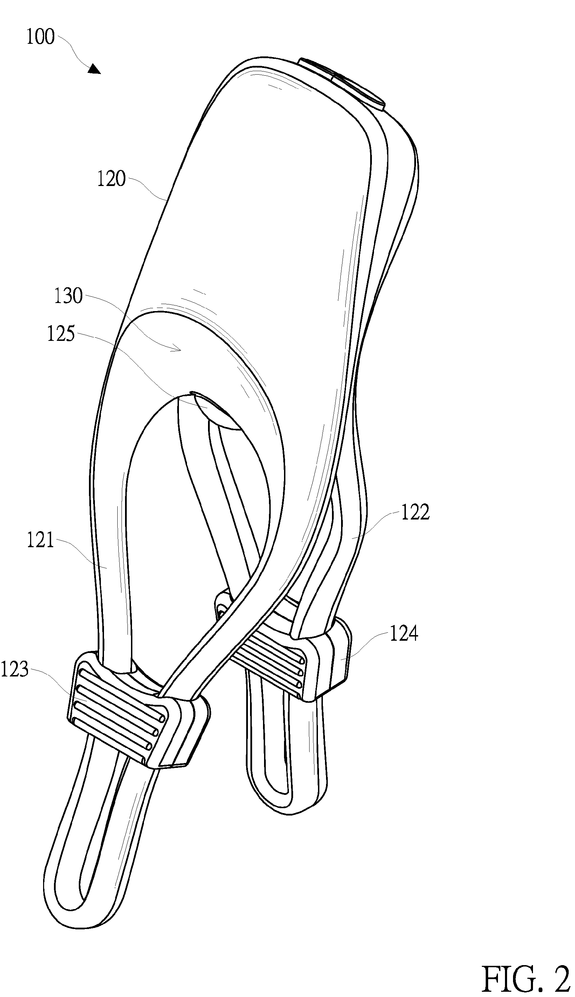

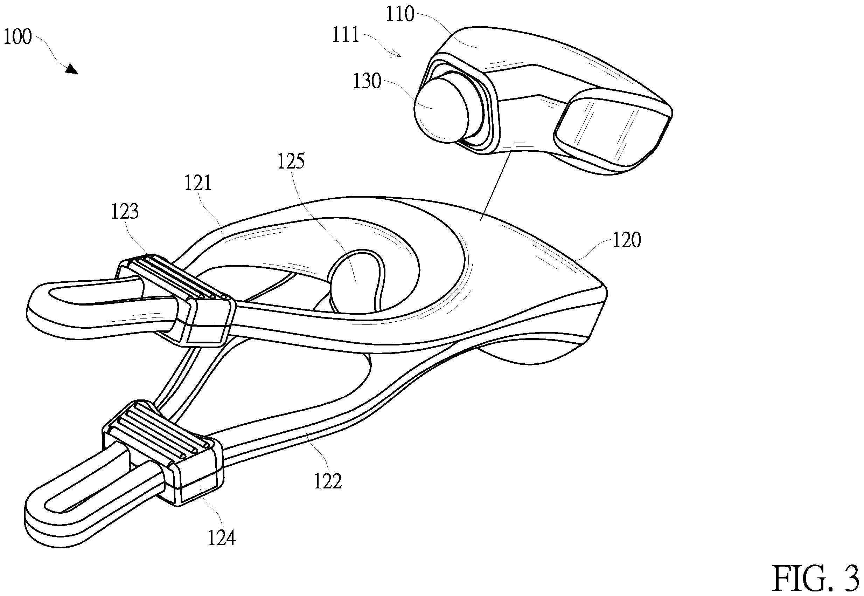

With reference to Figs. 2, 3, and 6, the penis measurement device 100 includes a main shell 110, an external soft casing 120, and a detection airbag 130. The main shell 110 includes a sensor opening 111. The detection airbag 130 is positioned inside of the main shell 110, and the detection airbag 130 protrudes outwards from the sensor opening 111 away from the main shell 110. The external soft casing 120 encases the main shell 110, and the external soft casing 120 includes at least one strap configured to be fixed on the penis. The penis measurement device 100 also includes a processing unit 10, a sensor chip 20, and a communication unit 30 that are mounted inside of the main shell 110. The sensor chip 20 contacts the detection airbag 130, the communication unit 30 is communicatively connected to the user device 200, and the processing unit 10 is respectively electrically connected to the sensor chip 20 and the communication unit 30.

In an embodiment, the at least one strap of the external soft casing 120 includes a first strap 121 and a second strap 122, as well as a first tightness regulator 123 corresponding to the first strap 121 and a second tightness regulator 124 corresponding to the second strap 122. The first strap 121 and the second strap 122 are both hollow polymer closed-loop straps. The first strap 121 and the second strap 122 are respectively connected to two opposite sides of the external soft casing 120 that correspond to a detection position 125 of the detection airbag 130. In addition, the first strap 121 is configured to pass through the first tightness regulator 123, and the first tightness regulator 123 may slide along the first strap 121 for regulating a tightness of the first strap 121. The second strap 122 is configured to pass through the second tightness regulator 124, and the second tightness regulator 124 may slide along the second strap 122 for regulating a tightness of the second strap 122. In other embodiments, the at least one strap of the external soft casing 120 may have different numbers of straps and strap configurations.

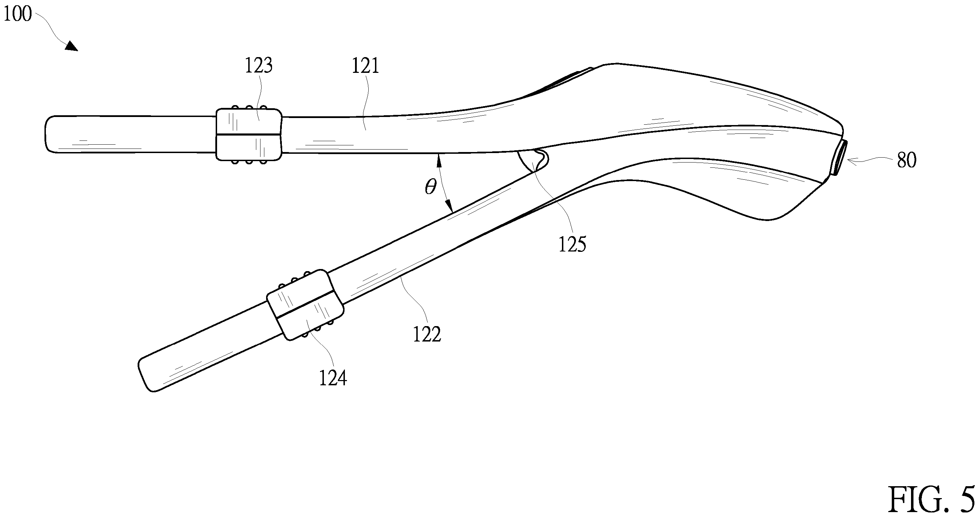

With reference to Figs. 5, 7, and 8, when the first strap 121 or the second strap 122 is tied to a penis 300, for example, when the penis measurement device 100 of the present invention is strapped to be on top of the penis 300 by the first strap 121, the second strap 122, the first tightness regulator 123 corresponding to the first strap 121, and the second tightness regulator 124 corresponding to the second strap 122, the main shell 110 is fixed to be on top of a root part 1 of the penis 300. Simultaneously, the detection airbag 130 contacts the penis 300 through the external soft casing 120, and the detection airbag 130, from top of the root part 1 of the penis 300, presses down onto the penis 300 with a down-pressing pressure

In an embodiment, the meaning of the detection airbag 130 contacting the penis 300 through the external soft casing 120, is that the detection airbag 130 extends outside of an opening of the external soft casing 120 to directly contact the penis 300. As such, the present invention is able to most simply and directly conduct measurements on the penis 300. In another embodiment, the meaning of the detection airbag 130 contacts the penis 300 through the external soft casing 120, is that the detection airbag 130 indirectly contacts the penis 300 through the external soft casing 120. As such, when conducting measurements on the penis 300, the external soft casing 120 is able to keep the sensor chip 20 inside of the main shell 110 dry from moisture and to keep the detection airbag 130 clean. In the present embodiment, the external soft casing 120 is completely waterproof. This allows the external soft casing 120 to be easily cleaned with water solutions and stay sanitized.

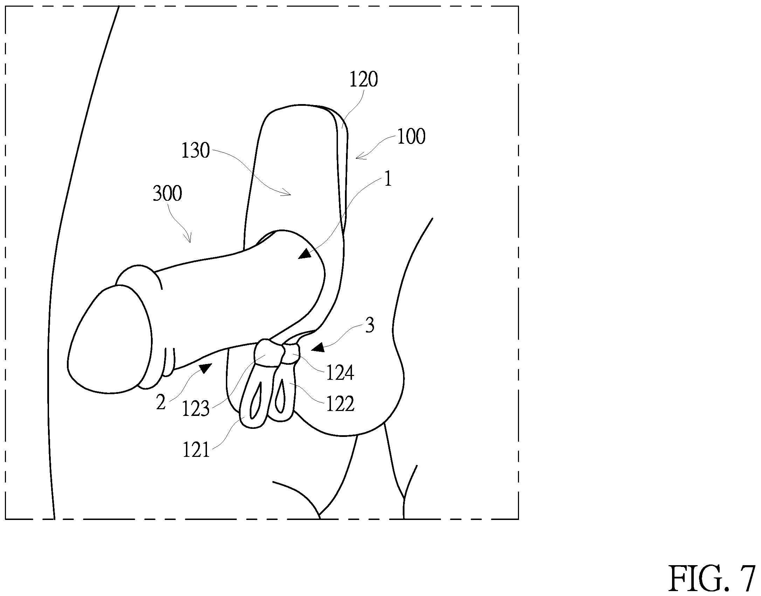

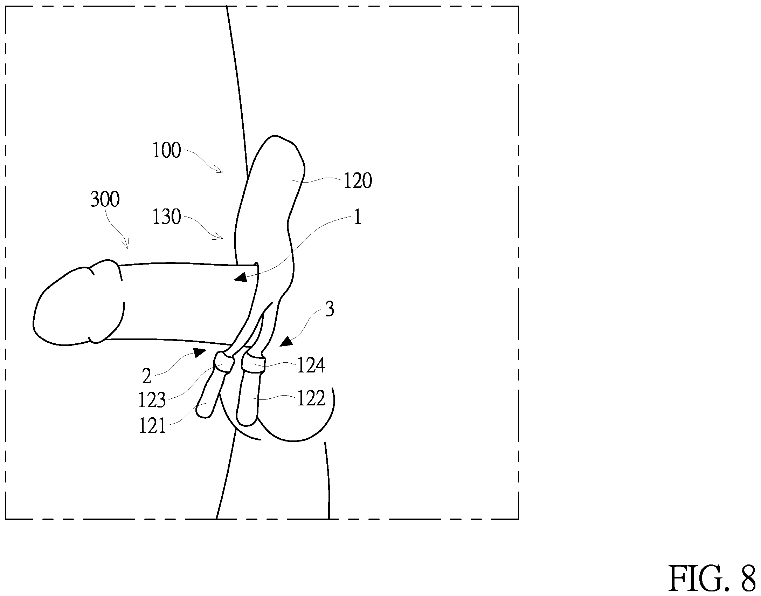

More particularly, the first strap 121 and the second strap 122 respectively extend towards two different directions from the two opposite sides of the detection position 125 on the external soft casing 120. The first strap 121 and the second strap 122, extending towards two different directions, form a difference angle θ in between. In an embodiment, the difference angle θ is 30 degrees. By having the difference angle θ optimized for the first strap 121 and the second strap 122 to tighten around the penis 300, the present invention is able to make sure that when the penis 300 is moving back and forth with the user's buttocks, i.e., when the user is having a sexual intercourse, the first strap 121 and the second strap 122 can be firmly fixed on the penis 300, ensuring that the penis measurement device 100 is free from being loose or falling from the root part 1 of the penis 300. By having the difference angle θ optimized for the first strap 121 and the second strap 122 to tighten around the penis 300, the present invention also avoids becoming an obstacle hindering the penis 300 from moving back and forth in motions. To ensure the penis measurement device 100 is securely fastened on the penis, when the first strap 121 and the second strap 122 are fastened on the penis 300, as shown in Fig. 7 and Fig. 8, the first strap 121 and the second strap 122 are respectively fastened by the first tightness regulator 123 and the second tightness regulator 124 below a bottom part 2 of the penis and above a top part 3 of a testicle/testicles. When the penis 300 is moving in back and forth motions, since the first tightness regulator 123 and the second tightness regulator 124 are fixed on the top part 3 of the testicles, the testicles would not be hurt, hindered, or disturbed by the first tightness regulator 123 and the second tightness regulator 124. In comparison to an apparatus of Rigiscan^{®}, the external soft casing 120 is able to structurally allow the detection airbag 130 to conduct measurement on the penis 300 without hindering the sexual intercourse process. Furthermore, the external soft casing 120 also prevents the penis measurement device 100 from falling during a man's sleep. As the external soft casing 120 is free from hurting the testicles, the man would be able to sleep comfortably.

About the detection airbag 130 conducting measurements on the penis 300, the detection airbag 130 contacting the penis on the root part 1 of the penis 300 is able to receive a bounce-back pressure corresponding to the down-pressing pressure from the penis 300. When the detection airbag 130 receives the bounce-back pressure corresponding to the down-pressing pressure, the detection airbag 130 deforms and squeezes (presses) onto the sensor chip 20, thus allowing the sensor chip 20 to generate a pressure sensor signal corresponding to a stress exerted onto the sensor chip 20 by the detection airbag 130. The sensor chip 20 then sends the pressure sensor signal to the processing unit 10, and the processing unit 10 calculates and outputs a hardness value according to the pressure sensor signal. The hardness value is the quantified quantity representing the hardness of the user's penis.

The harder the penis 300 is, the less the penis would deform when receiving the down-pressing pressure, hence the penis 300 would produce the greater the bounce-back pressure corresponding to the down-pressing pressure. As the detection airbag 130 receives the greater the bounce-back pressure, the detection airbag 130 would deform more to squeeze more onto the sensor chip 20, thus putting more stress onto the sensor chip 20. As the sensor chip 20 receives the greater stress, the sensor chip 20 outputs the greater pressure sensor signal to the processing unit 10, thus allowing the processor unit 10 to calculate the greater hardness value.

Vice versa, the softer the penis 300 is, the greater the penis 300 would deform when receiving the down-pressing pressure, hence the penis 300 would produce the lesser bounce-back pressure corresponding to the down-pressing pressure. As the detection airbag 130 receives the lesser bounce-back pressure, the detection airbag 130 would deform less to squeeze less onto the sensor chip 20, thus putting lesser stress onto the sensor chip 20. As the sensor chip 20 receives the lesser stress, the sensor chip 20 outputs the lesser pressure sensor signal to the processing unit 10, thus allowing the processor unit 10 to calculate the lesser hardness value.

As the apparatus of Rigiscan^{®} is not completely accurate in measuring the hardness and the enlargement of the penis, for example, as described in prior art that the Rigiscan^{®} is only able to measure up to roughly 60% of the hardness of the enlarged penis, the present invention uses a completely different measuring principle. The present invention utilizes a hardness measuring principle used by a Shore durometer, thus measuring the hardness of the penis with better accuracy, in other words, more accurately gauging the bounce-back ability of the penis after the penis endures the down-pressing pressure.

The hardness value of the present invention is originated from the pressure sensor signal generated and outputted by the sensor chip 20. In the present embodiment, the sensor chip 20 is a piece of micro electro mechanical systems (MEMS) configured for detecting pressure. For example, the sensor chip 20 may be a pressure sensor of model LPS22HBTR manufactured by Mouser Electronics^{®}. The said pressure sensor uses a strain gauge as a pressure sensitive component, and the stress sensed by the strain gauge is the pressure forced onto the sensor chip 20 by the detection airbag 130. Although the hardness value of the present invention originated from the pressure changes sensed by the sensor chip 20, when the sensor chip 20 is structurally configured with the rest of components in the penis measurement device 100, the hardness value calculated by the present invention retains the physical meaning of quantifying the hardness of the penis. The hardness value calculated by the present invention and value gauged by Shore hardness are positively correlated.

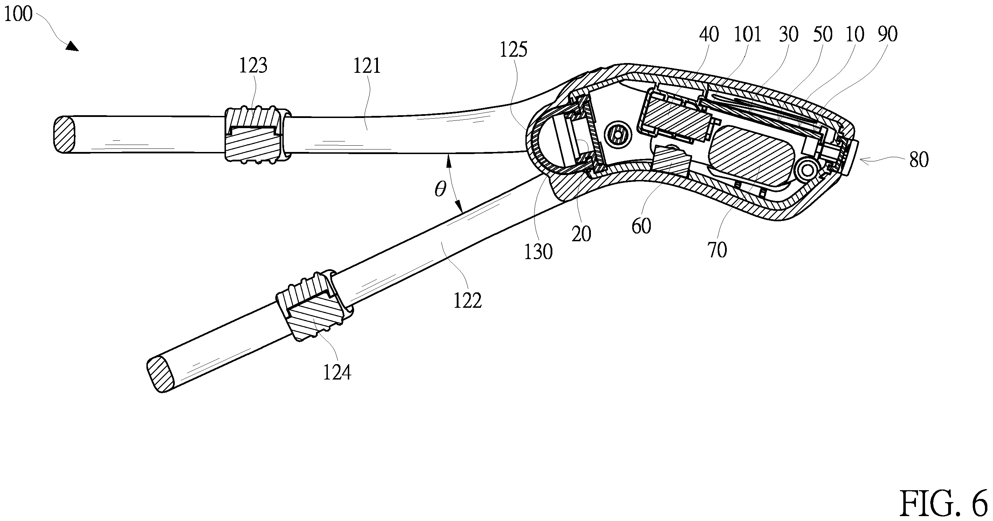

Furthermore, in an embodiment, the penis measurement device 100 further includes an eccentric rotating mass (ERM) motor 40, a gyroscope 50, a charging port 60, a battery unit 70, a buttons unit 80, and a memory unit 90. The ERM motor 40, the gyroscope 50, the charging port 60, the battery unit 70, the buttons unit 80, and the memory unit 90 are respectively electrically connected to the processing unit 10, and the charging port 60 is further electrically connected to the battery unit 70. The ERM motor 40, the gyroscope 50, and the battery unit 70 are mounted inside of the main shell 110, and the charging port 60 and the buttons unit 80 are mounted on the main shell 110. With reference to Fig. 6, in the present invention, the penis measurement device 100 further includes a circuit board 101. The processing unit 10, the communication unit 30, the gyroscope 50, and the memory unit 90 are all mounted on the circuit board 101. The processing unit 10 is a processor, and the memory unit 90 is a memory.

When the ERM motor 40 is started by the processing unit 10, the ERM motor 40 produces vibrations, thus vibrating the penis measurement device 100.

The gyroscope 50 generates a gyroscope signal according to movement changes of the penis measurement device 100 and sends the gyroscope signal to the processing unit 10. The processing unit 10 calculates a gyroscope value according to the gyroscope signal. The meaning of the gyroscope value will be explained in later parts of the description. In general, regardless of the user wearing the penis measurement device 100 wearing any kinds of conscious or unconscious movements relating to the penis 300, such as having the sexual intercourse or the NPT, the gyroscope 50 would be able to record movement and directional changes of the penis measurement device 100 into the gyroscope signal, and send the gyroscope signal to the processing unit 10 for the processing unit 10 to calculate the gyroscope value that is invaluable for analyzing physiological status of the user. By having the gyroscope 50, the back and forth motions of the buttocks of the user may be additionally monitored during the sexual intercourse.

After calculating the hardness value and the gyroscope value, the processing unit 10 may control the communication unit 30 for communicatively connecting to the user device 200, thus having the communication unit 30 sending the hardness value and the gyroscope value to the user device 200. In an embodiment, the communication unit 30 of the penis measurement device 100 uses Bluetooth^{®} to communicatively connect to the user device 200. As the user device 200 receives the hardness value and the gyroscope value from the penis measurement device 100, the user device 200 may conduct further calculations and data processing procedures using its own settings, thus providing the user with additional information regarding the penis of the user. The user may examine the data collected by the penis measurement device 100 through the user device 200. In an embodiment, when the processing unit 10 controls the ERM motor 40 to produce vibrations, since the vibration emanating from the ERM motor 40 does not significantly displace the penis measurement device 100, the gyroscope 50 would still be able to count the back and forth motions of the penis measurement device 100 unaffected.

The battery unit 70 stores electrical charges for the penis measurement device 100. The battery unit 70 may be rechargeable batteries, and the charging port 60 is configured to electrically connect an external power source for charging the battery unit 70.

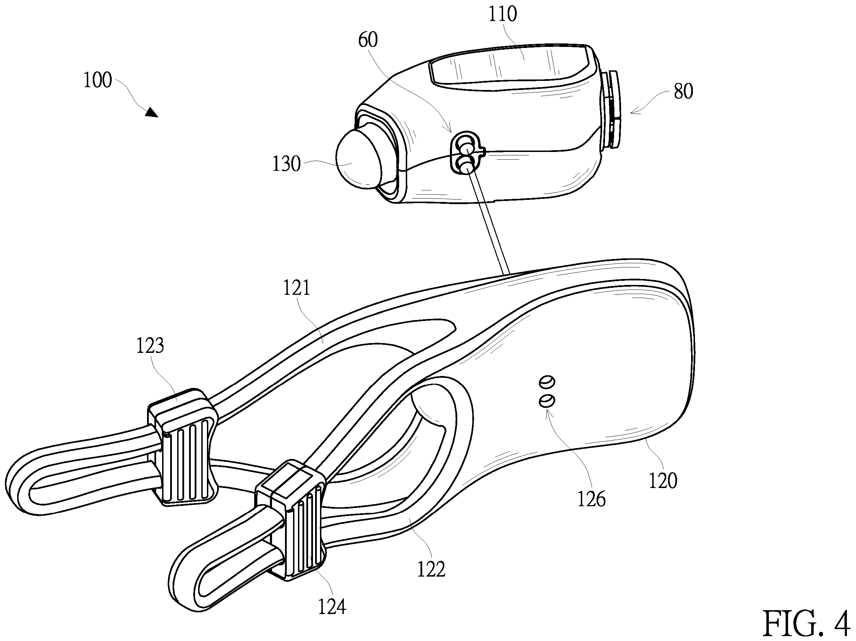

With further reference to Fig. 4, the external soft casing 120 includes a hole 126, and the hole 126 corresponds to a position of the charging port 60. When the external soft casing 120 encases the main shell 110, the charging port 60 on the main shell 110 is configured to position in the hole 126, thus allowing the charging port 60 to be exposed for charging.

In an embodiment, the user device 200 is a computer system, and therefore, the user device 200 includes a system unit, a memory, a display, a keyboard, a mouse, and a module responsible for communicating with the communication unit 30 of the penis measurement device 100. The user device 200 and the communication unit 30 of the penis measurement device 100 may be connected through physical ports and hard-wires, such as connected through Ethernet ports or universal serial bus (USB) ports.

In another embodiment, the user device 200 is a smart personal device, such as a smart phone, a tablet computer, or a smart wearable device. In the present embodiment, the user device 200 is a smart phone, thus the user device 200 includes a touch screen, a memory, and a wireless communication module. The user device 200 uses the wireless communication module to communicate with the communication unit 30 of the penis measurement device 100. In the embodiment, the touch screen is the display of the user device 200. The memory of the user device 200 stores a plurality of mode options, and the user may select one of the mode options to control the penis measurement device 100. The user device 200 may also connect to a cloud server through the communication unit 30, allowing the user device 200 to upload and store the physiological data measured from the penis measurement device 100.

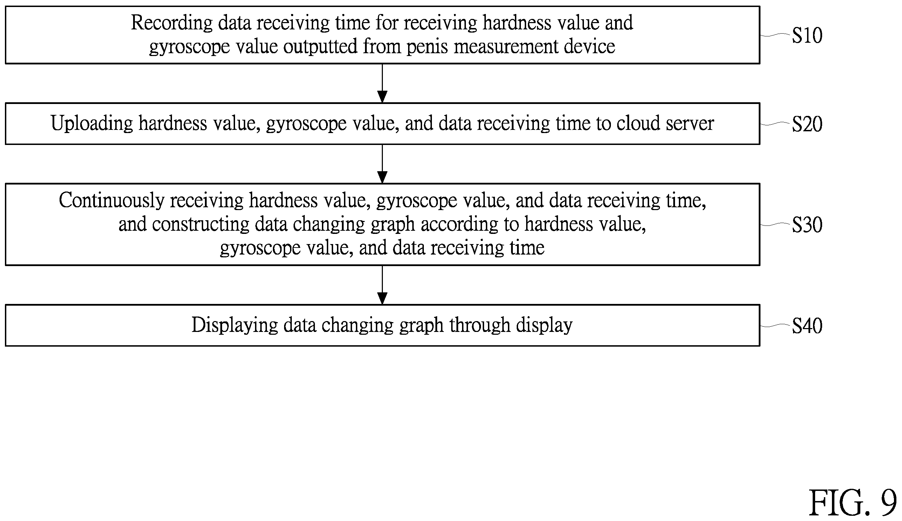

With reference to Fig. 9, the present invention also provides a penis measurement method. The penis measurement method is executed by a user device of the penis measurement system. The penis measurement method includes the following steps:
step S10: recording a data receiving time for receiving a hardness value and a gyroscope value outputted from a penis measurement device;
step S20: uploading the hardness value, the gyroscope value, and the data receiving time to a cloud server;
step S30: continuously receiving the hardness value, the gyroscope value, and the data receiving time, and constructing a data changing graph according to the hardness value, the gyroscope value, and the data receiving time; and
step S40: displaying the data changing graph through a display.

In an embodiment, the data receiving time is recorded according to a system time of the user device 200 at the moment the user device 200 receives the hardness value and the gyroscope value.

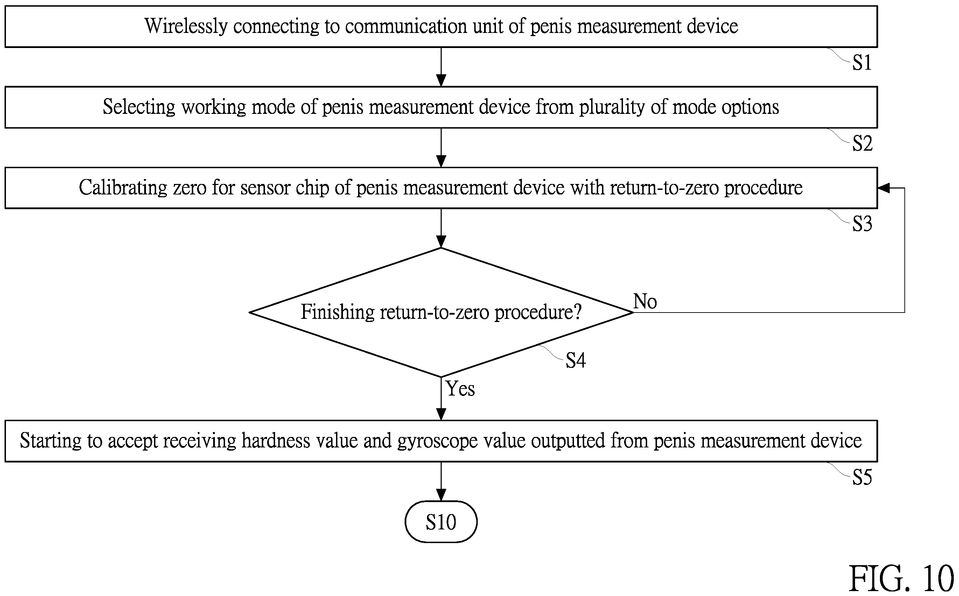

With reference to Fig. 10, in an embodiment, before step S10, the penis measurement method further includes the following steps:
step S1: wirelessly connecting to the communication unit of the penis measurement device;
step S2: selecting a working mode of the penis measurement device from the plurality of mode options;
step S3: calibrating zero for a sensor chip of the penis measurement device with a return-to-zero procedure;
step S4: determining whether finishing return-to-zero procedure; when the return-to-zero procedure is unfinished, executing step S3; and
step S5: when the return-to-zero procedure is finished, starting to accept receiving the hardness value and the gyroscope value outputted from the penis measurement device, and further executing step S10.

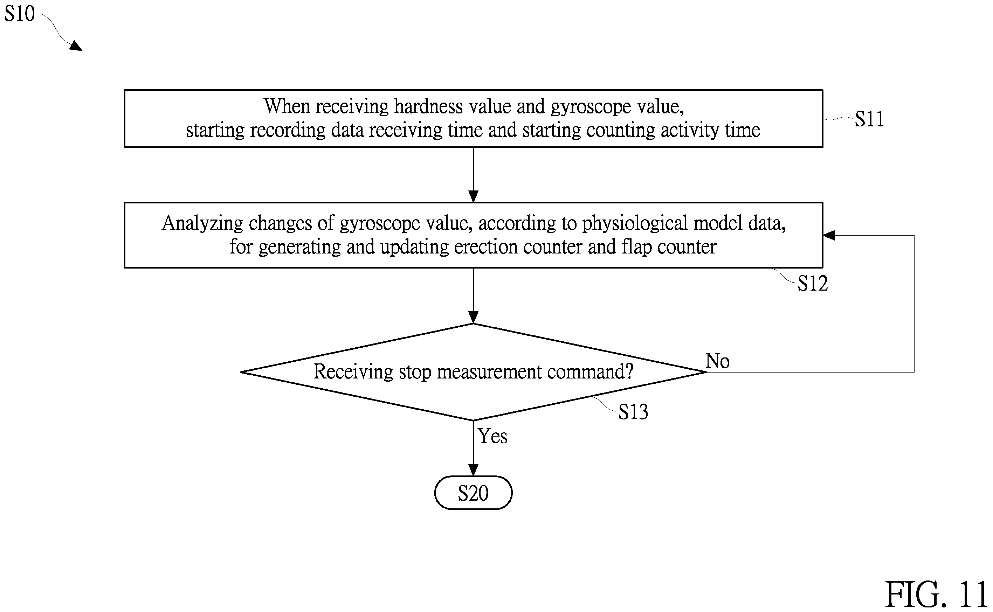

With reference to Fig. 11, in an embodiment, the step S10 further includes the following sub-steps:
step S11: when receiving the hardness value and the gyroscope value, starting recording the data receiving time and starting counting an activity time;
step S12: analyzing changes of the gyroscope value, according to a physiological model data, for generating and updating an erection counter and a flap counter; and
step S13: determining whether receiving a stop measurement command; when without receiving the stop measurement command, executing step S12; when receiving the stop measurement command, executing step S20.

When executing step S20, the present invention may also execute the following step: uploading the erection counter and the flap counter accumulated by the user device to the cloud server, and displaying the erection counter, the flap counter, and the activity time on the display of the user device.

In the present embodiment, the aforementioned user device 200 is a smart phone, and the smart phone uses an application (APP) to execute the penis measurement method of the present invention for controlling the penis measurement device 100. With reference to the aforementioned descriptions, the following examples are demonstrated in tandem with perspective views of screenshots of software interfaces from the APP that is executed by the smart phone.

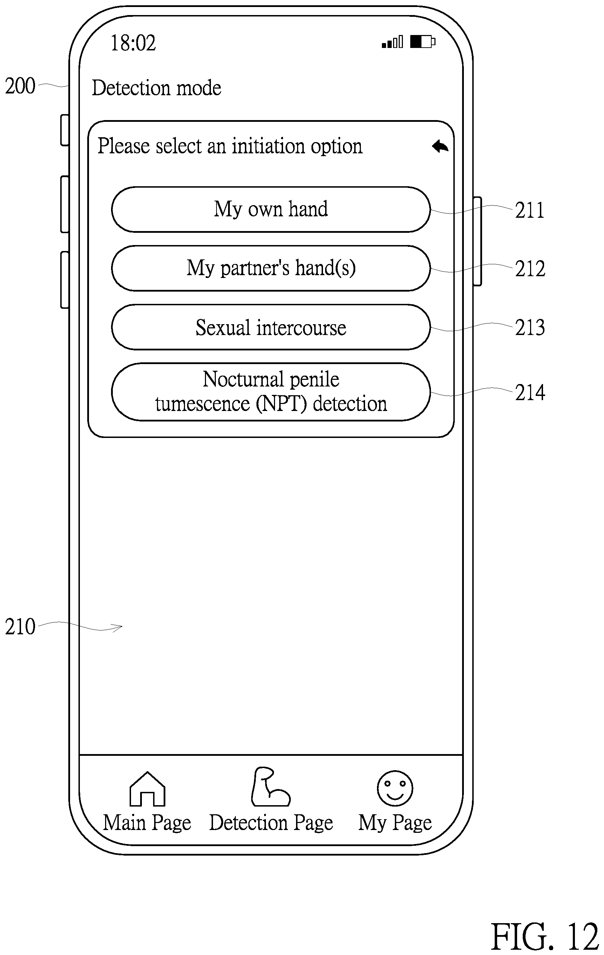

With reference to Fig. 12, the user is able to look at the information presented by the APP displayed on the touch screen of the user device 200 and use the touch screen to select a working mode from a plurality of mode options in the APP. In an embodiment, on a first option page 210 of the APP that is displayed by the touch screen of the user device 200, multiple options are presented to the user for choosing an initiation option of the penis measurement device 100, thus configuring the penis measurement device 100 to work in tandem with the user. Through various pages of the APP, displayed by the touch screen of the user device 200, mainly the user device 200 is able to provide the user with a data result of the measurement on the penis. The data result can then be subsequently used by the user or professionals for evaluation and analysis. For example, the mode options provided to the user through the APP for selecting the initiation option includes a participation with my own hand option 211, a participation with my partner's hand(s) option 212, a partake in sexual intercourse option 213, and a partake in NPT detection option 214. The initiation option selected by the user would influence parameters of how the user device 200 controls the penis measurement device 100 to conduct measurements of the penis.

In an embodiment, when the partake in NPT detection option 214 is selected as the initiation option, and once the penis measurement device 100 communicates to the user device 200 that the penis measurement device 100 is ready for conducting measurements, the user device 200 would disconnect its Bluetooth connection from the penis measurement device 100. After being communicatively disconnected from the user device 200, the penis measurement device 100 would store the measurements, i.e. the hardness value and the gyroscope value, of the penis over a period of time in the memory unit 90. When the user wakes up the next morning, and when the user uses the user device 200, such as the smart phone, to communicatively re-connect with the penis measurement device 100, the penis measurement device 100 would send the hardness value and the gyroscope value accumulated over the period of time in the memory unit 90 to the user device 200 via Bluetooth connection. Subsequently, the smart phone would then upload the hardness value and the gyroscope value for the period of time to a database of the cloud server. As such, physiological data of monitoring the penis over a night is recorded and uploaded to the cloud server online. More particularly, the period of time monitoring the hardness value and the gyroscope value, refers to a time when the penis measurement device 100 starts taking data to a time when the user wakes up and switches off the penis measurement device 100. When the penis measurement device 100 receives a switch off command and prepares to shut down, the penis measurement device 100 stops recording the hardness value and the gyroscope value. When the penis measurement device 100 is subsequently switched on, the penis measurement device 100 then proceeds to send the hardness value and the gyroscope value gathered over the period of time to the user device 200.

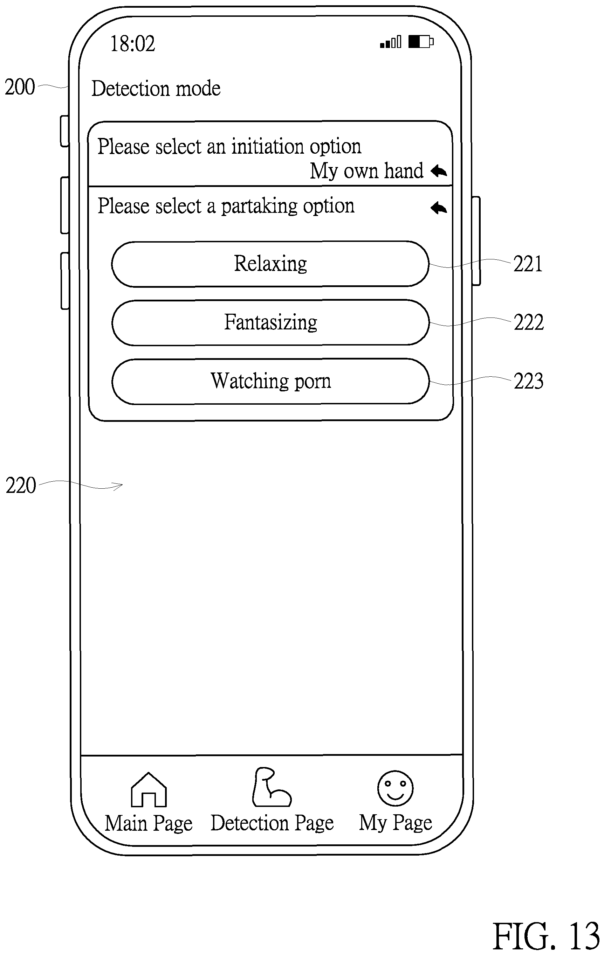

With reference to Fig. 13, after the participation with my own hand option 211 is selected for the initiation option, the APP of the user device 200 displays a second option page 220 through the touch screen. The mode options provided to the user for selecting a partaking option includes a partake in relaxation option 221, a partake in fantasizing option 222, and a partaking watching porn option 223. The partaking option selected by the user provides the professionals, such as sex therapist, with invaluable evidence for analyzing data and subsequently guiding the user to improve sexual activities and physiological status.

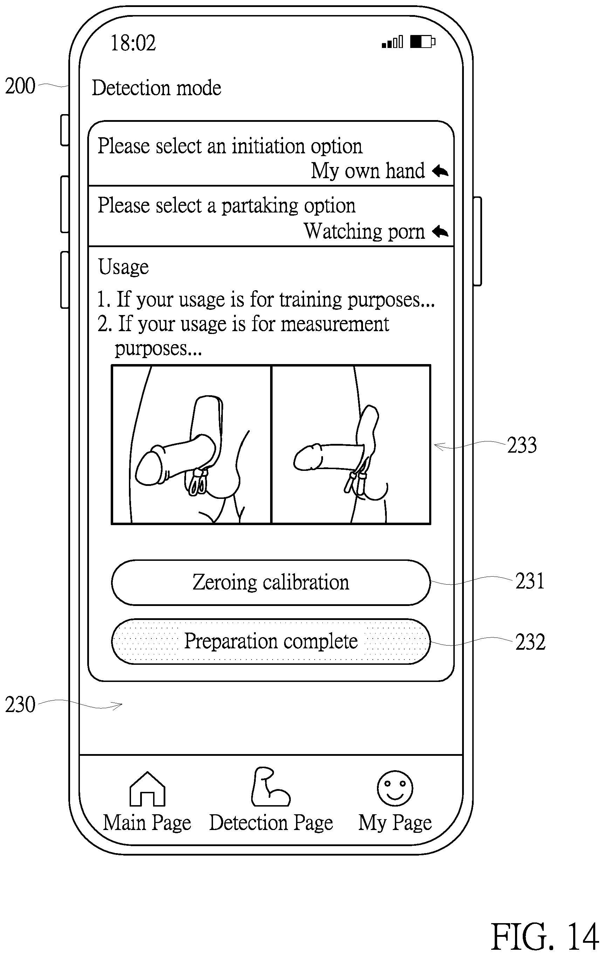

With reference to Fig. 14, after the partaking watching porn option 223 is selected for the partaking option, the APP of the user device 200 displays a calibration to zero page 230 through the touch screen. The calibration to zero page 230 includes a zeroing calibration option 231 and a preparation complete option 232. Simultaneously, the calibration to zero page 230 also includes a wearing instruction information 233 for guiding the user to wear the penis measurement device 100. When the zeroing calibration option 231 is selected, the user device 200 thus acknowledges that the user has put on the penis measurement device 100 by following the wearing instruction information 233, and the user device 200 proceeds to control the penis measurement device 100 for calibration with the aforementioned return-to-zero procedure.

When the return-to-zero procedure is still on-going, the preparation complete option 232 on the calibration to zero page 230 is thus still unavailable for the user to select. When the return-to-zero procedure is completed, only then does the user device 200 make the preparation complete option 232 on the calibration to zero page 230 available for the user to select. For example, in the example shown in Fig. 14, the preparation complete option 232 covered in dots symbolizes that the preparation complete option 232 is still unavailable for selection. When the preparation complete option 232 is available for selection, then the preparation complete option 232 would be spotless as shown for the zeroing calibration option 231 on the calibration to zero page 230. When the preparation complete option 232 is selected, the user device 200 thus acknowledges that the user has completed all preparations and is ready for measurements on the penis, and the user device 200 proceeds to control the penis measurement device 100 for conducting the measurements on the hardness of the penis and gather other physiological data from the user. The other physiological data, for example, as previously disclosed, may be obtaining the erection counter and the flap counter by analyzing changes of the gyroscope value according to the physiological model data stored in the user device 200.

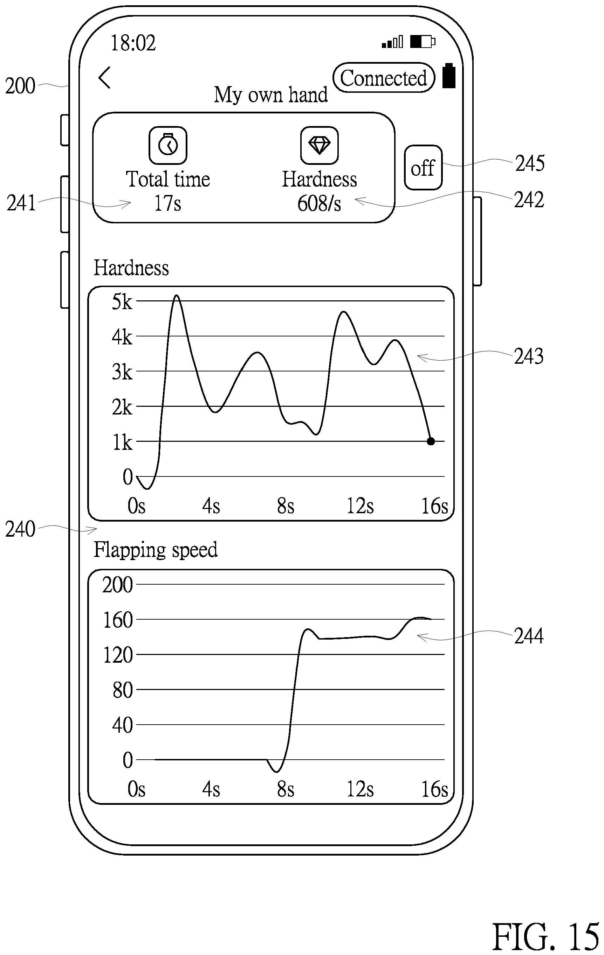

With reference to Fig. 15, after the preparation complete option 232 is selected, the penis measurement device 100 starts delivering the hardness value and the gyroscope value to the user device 200 in real-time. The user device 200 thus starts obtaining real-time measurements of the hardness value and the gyroscope value taken by the penis measurement device 100, and the APP of the user device 200 displays a real-time data page 240 through the touch screen. The user device 200 executes the aforementioned steps of the penis measurement method for displaying an activity time 241, a hardness value 242, a hardness data changing graph 243 corresponding to changes of the hardness value 242 over time, and a flapping speed changing graph 244 corresponding to changes of the gyroscope value over time on the real-time data page 240. In the example shown in Fig. 15, the hardness value 242 is the hardness value measured by the senor chip 20 over each second. In other words, the hardness value 242 has a denominator as one second (1/second, or 1/s), and the hardness value 242 is measured as the hardness value/s. A vertical axis of the hardness data changing graph 243 represents the hardness value 242, and a horizontal axis of the hardness data changing graph 243 represents chronological progression of the activity time 241. A vertical axis of the flapping speed changing graph 244 records a flapping speed. The flapping speed, for example, is measured as the gyroscope value per second, thus may be represented as (the gyroscope value)/s. A horizontal axis of the flapping speed changing graph 244 also represents chronological progression of the activity time 241. As the activity time 241 and the hardness value 242 are updated in real-time, the hardness data changing graph 243 and the flapping speed changing graph 244 are also continuously being updated. The vertical axis and horizontal axis of both the hardness data changing graph 243 and the flapping speed changing graph 244 would be continuously adjusted for optimizing scales presented to the user. The continuous update and adjustments of the hardness data changing graph 243 and the flapping speed changing graph 244 present immediate physiological feedbacks to the user while the user is having sexual activities with the penis. In general, towards different degrees and ways of back and forth body movements, or towards different degrees and ways of flapping, the present invention would measure and analyze the hardness value and the gyroscope value differently. The present invention does not limit the ways of measuring and analyzing the hardness value and the gyroscope value of the present invention. The emphasis of the present invention, is that through parameters configured by the APP, the sensor chip 20 and the gyroscope 50 of the penis measurement device 100 may be programmed to measure the hardness value and the gyroscope value differently. In an embodiment, the hardness value is X/hPa (wherein X is the numeric measured value, and hPa is a pressure unit of a hundred Pascal). In an embodiment, the gyroscope value may be measured in a numeric count of back and forth motion over a minute or over a second, or in other words, (the numeric count of a flap)/min or (the numeric count of a flap)/s. The numeric count of a flap is the flap counter.

In an embodiment, the real-time data page 240 also displays a stop option 245. When the stop option 245 is selected, the user device 200 immediately generates the stop measurement command, thus the user device 200 stops the penis measurement device 100 from measuring the penis according to the stop measurement command.

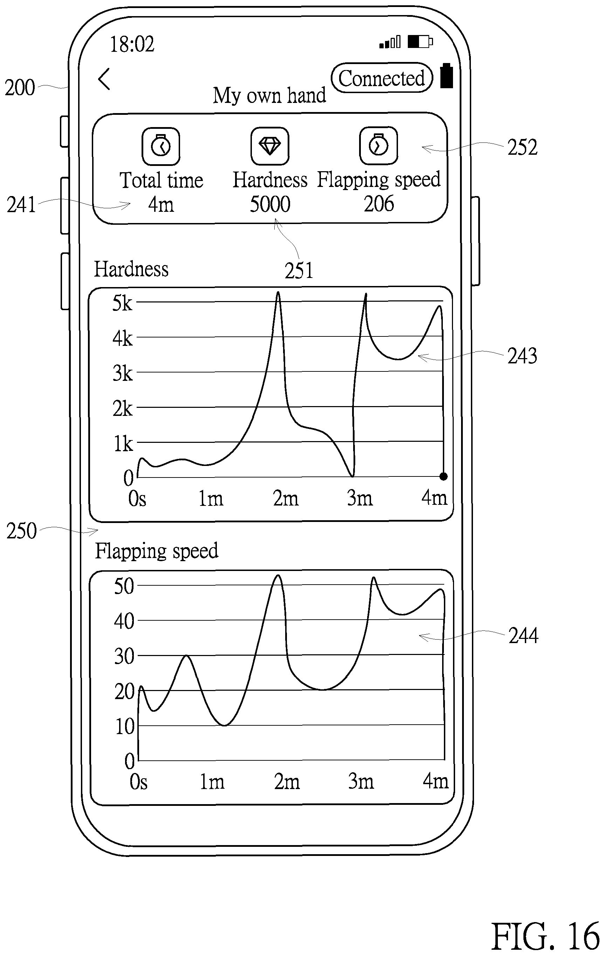

With reference to Fig. 16, once stopping measuring, the APP of the user device 200 displays a data result page 250 through the touch screen. The data result page 250 retains the most recent update of the activity time 241, the hardness data changing graph 243, and the flapping speed changing graph 244. The user device 200 further executes the following steps:
recording a total time as the activity time 241 last updated by the user device 200, and displaying the total time;
obtaining a highest value of the vertical value of the hardness data changing graph 243 across all times, setting the highest value as a highest hardness value, and displaying the highest hardness value;
calculating a total peak number by counting all the peaks of the vertical values of flapping speed changing graph 244 that are greater than a threshold, setting the total peak number as a numeric count of a flap, and displaying the numeric count of the flap, or in other words, displaying the flap counter.

For example, corresponding to the above steps, a highest hardness value 251 and a numeric count 252 of a flap are displayed on the data result page 250.

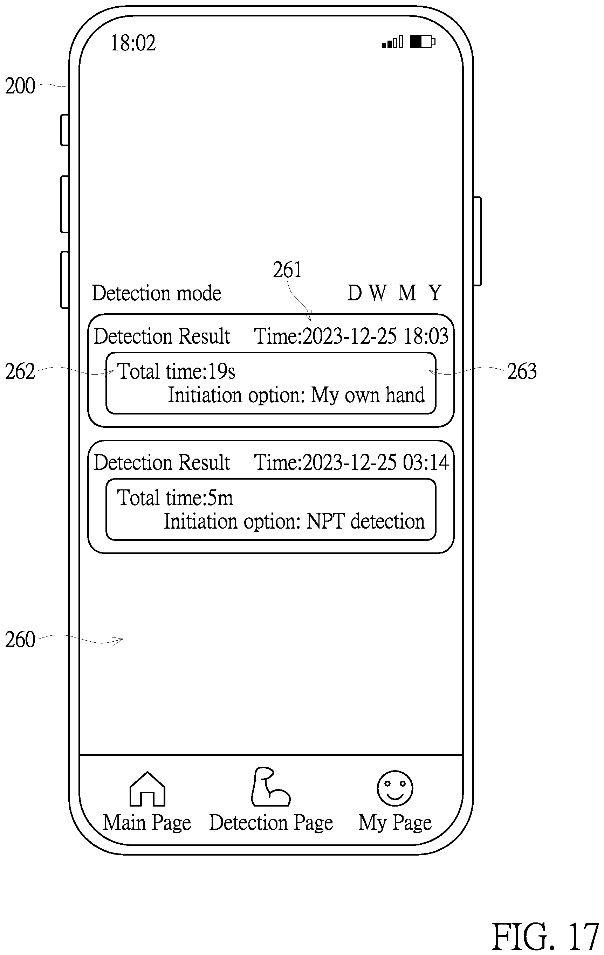

With reference to Fig. 17, the touch screen of the user device 200 may, at any given time, be switched to display a historical records page 260 of the APP. The historical records page 260 displays historical records of the user's physiological data stored in the database of the cloud server. Each record of the user's physiological data on the historical records page 260 includes a record time 261, a total time 262, and a mode information 263. The record time 261 is obtained from a central time synchronized by the smart phone and the cloud server, and the record time 261 is also the aforementioned data receiving time recorded by the penis measurement device 100 for receiving hardness value and gyroscope value. The total time 262 is the aforementioned total time for recording the said record of the user's physiological data, in other words, the total time 262 is the activity time 241 last updated for the said record of the user's physiological data. The mode information 263 corresponds to the initiation option chosen by the user through the user device 200 for configuring how the penis measurement device 100 should conduct measurements of the penis. Each record of the user's physiological data on the historical records page 260 may be selected by the user through interacting with the touch screen for revealing more detailed recorded physiological information.

Overall, the user of the present invention is able to obtain unbiased, scientific, and quantitative physiological information of the penis under various application settings through wearing the penis measurement device 100. The physiological information of the penis gathered by the present invention, for example, may include the hardness value of the penis when the user is having a sexual intercourse. Furthermore, the present invention provides the user an easy convenience of accessing the historical records of the user's physiological data measurements through the user device 200 accessing the database of the cloud server. For example, the user may access to read about the highest hardness value 251 of the penis that is analyzed for a training practice of the penis or a sexual intercourse of the penis. These invaluable data are able to assist the user oneself, doctors, or professionals to more effectively, more accurately, and more scientifically measure the hardness of the user's penis, thus providing invaluable insights over the user's physiological status, and providing assistive information for the doctors or the professionals to decide on further treatments or training courses related to the penis for the user.

For reference, please consider the following research papers:
Research paper 1: Ku, J., Song, Y, Kim, M., Lee, N., & Park, Y. (2001). "Is there a role of radial rigidity in the evaluation of erectile dysfunction?" International journal of impotence research, 13(4), 200-204; and
Research paper 2:Rohrer, G. E., Premo, H., & Lentz, A. C. (2022). "Current Techniques for the Objective Measures of Erectile Hardness." Sexual Medicine Reviews, 10(4), 648-659.

According to the research papers 1 and 2, the research papers 1 and 2 support that the hardness of the penis along a radial direction and a resistive force along the radial direction are significantly correlated, and thus the hardness value of the penis that is measured by the present invention is indeed positively correlated to how much resistance force is measured by the present invention. The measurement obtained by the present invention is able to help further investigating the physiological research of: exactly how hard the penis needs to be for the penis to successfully overcome the resistive force imposed by the vagina in order for the penis to successfully enter into the vagina.

## Claims

1. A penis measurement system, **characterized in that** the penis measurement system comprises:
a penis measurement device (100), configured to be fixed on a penis (300) of a user, and comprising:
a main shell (110), comprising a sensor opening (111);
a detection airbag (130), positioned inside of the main shell (110), and protruding outwards from the sensor opening (111);
a sensor chip (20), mounted inside of the main shell (110), and contacting the detection airbag (130);
a communication unit (30), mounted inside of the main shell (110);
a processing unit (10), mounted inside of the main shell (110), and electrically connected to the sensor chip (20) and the communication unit (30);
an external soft casing (120), encasing the main shell (110), and comprising at least one strap configured to be fixed on the penis (300);
wherein when the at least one strap is strapped on the penis (300), the external soft casing (120) fixes the main shell (110) to be on top of a root part (1) of the penis (300), the detection airbag (130) contacts the penis (300) through the external soft casing (120), and the detection airbag (130), from top of the root part (1) of the penis (300), presses down onto the penis (300) with a down-pressing pressure;
wherein when the detection airbag (130) receives a bounce-back pressure corresponding to the down-pressing pressure from the penis (300), the detection airbag (130) deforms and presses onto the sensor chip (20), thus allowing the sensor chip (20) to generate a pressure sensor signal corresponding to a stress exerted onto the sensor chip (20) by the detection airbag (130); the sensor ship sends the pressure sensor signal to the processing unit (10), and the processing unit (10) calculates and outputs a hardness value according to the pressure sensor signal;
a user device (200), communicatively connected to the communication unit (30) of the penis measurement device (100); wherein the user device (200) receives the hardness value outputted by the processing unit (10) via the communication unit (30) of the penis measurement device (100);
a gyroscope (50), mounted inside of the main shell (110), and electrically connected to the processing unit (10); wherein the gyroscope (50) generates a gyroscope signal according to movement changes of the penis measurement device (100) and sends the gyroscope signal to the processing unit (10);
wherein the user device (200) calculates a gyroscope value according to the gyroscope signal, and the processing unit (10) controls the communication unit (30) to send the gyroscope value to the user device (200).

2. The penis measurement system as claimed in claim 1, wherein the at least one strap of the external soft casing (120) comprises a first strap (121) and a second strap (122); the first strap (121) and the second strap (122) are hollow polymer closed-loop straps; the first strap (121) and the second strap (122) are respectively connected to two opposite sides of the external soft casing (120) that correspond to a detection position (125) of the detection airbag (130);
wherein the first strap (121) and the second strap (122) respectively extend towards two different directions from the two opposite sides of the detection position (125) on the external soft casing (120);
wherein when the first strap (121) and the second strap (122) are fastened on the penis (300), the first strap (121) and the second strap (122) are fastened below a bottom part (2) of the penis (300) and above a top part (3) of a testicle/testicles.

3. The penis measurement system as claimed in claim 2, wherein the at least one strap of the external soft casing (120) also comprises a first tightness regulator (123) corresponding to the first strap (121) and a second tightness regulator (124) corresponding to the second strap (122);
wherein the first strap (121) is configured to pass through the first tightness regulator (123), and the first tightness regulator (123) slides along the first strap (121) for regulating a tightness of the first strap (121);
wherein the second strap (122) is configured to pass through the second tightness regulator (124), and the second tightness regulator (124) slides along the second strap (122) for regulating a tightness of the second strap (122).

4. The penis measurement system as claimed in claim 1, wherein the penis measurement device (100) further comprises:
a battery unit (70), mounted inside of the main shell (110), electrically connected to the processing unit (10), and storing electrical charges;
a charging port (60), mounted on the main shell (110), and electrically connected to the processing unit (10) and the battery unit (70); wherein the external soft casing (120) comprises a hole (126), and the hole (126) corresponds to a position of the charging port (60); when the external soft casing (120) encases the main shell (110), the charging port (60) on the main shell (110) is configured to position in the hole (126);
an eccentric rotating mass (ERM) motor (40), mounted inside of the main shell (110), and electrically connected to the processing unit (10); wherein when the ERM motor (40) is started by the processing unit (10), the ERM motor (40) produces vibrations.

5. The penis measurement system as claimed in claim 1, wherein the sensor chip (20) is a piece of micro electro mechanical systems (MEMS) configured for detecting pressure.

6. The penis measurement system as claimed in claim 1, wherein the user device (200) is a smart phone, and the smart phone is wirelessly connected to the communication unit (30) of the penis measurement device (100);
wherein the smart phone executes an application (APP) with a software interface for controlling the penis measurement device (100).

7. The penis measurement system as claimed in claim 6, wherein the penis measurement device (100) comprises a memory unit (90), and the memory unit (90) is electrically connected to the processing unit (10);
wherein when a partake in nocturnal penile tumescence (NPT) detection option (214) is selected through the software interface of the APP, and once the penis measurement device (100) communicates to the user device (200) that the penis measurement device (100) is ready for conducting measurements, the user device (200) disconnects from the penis measurement device (100), and the penis measurement device (100) stores the hardness value and the gyroscope value over a period of time in the memory unit (90);
wherein when the user device (200) communicatively re-connects with the penis measurement device (100), the penis measurement device (100) sends the hardness value and the gyroscope value accumulated over the period of time in the memory unit (90) to the user device (200) wirelessly.

8. The penis measurement system as claimed in claim 6, wherein the software interface of the APP simultaneously presents a zeroing calibration option (231), a preparation complete option (232), and an instruction information (233) for guiding the user to wear the penis measurement device (100);
wherein when the zeroing calibration option (231) is selected, the user device (200) proceeds to calibrate the penis measurement device (100);
wherein after the user device (200) calibrates the penis measurement device (100), when the preparation complete option (232) is selected, the user device (200) proceeds to control the penis measurement device (100) for taking measurements on the penis (300).

9. The penis measurement system as claimed in claim 6, wherein the penis measurement device (100) delivers the hardness value and the gyroscope value to the user device (200) in real-time;
wherein the user device (200) displays a graphical representation of changes of the hardness value in real-time through the APP.

10. A penis measurement method, **characterized in that** the penis measurement method is executed by the user device (200) as claimed in claim 1 and comprises the following steps:
recording a data receiving time for receiving a hardness value and a gyroscope value outputted from a penis measurement device (100);
uploading the hardness value, the gyroscope value, and the data receiving time to a cloud server;
continuously receiving the hardness value, the gyroscope value, and the data receiving time, and constructing a data changing graph according to the hardness value, the gyroscope value, and the data receiving time; and
displaying the data changing graph through a display.

11. The penis measurement method as claimed in claim 10, wherein the step of recording the data receiving time for receiving the hardness value and the gyroscope value comprises the following sub-steps:
when receiving the hardness value and the gyroscope value, starting recording the data receiving time and starting counting an activity time;
analyzing changes of the gyroscope value, according to a physiological model data stored in the user device (200), for generating and updating an erection counter and a flap counter; and
when receiving a stop measurement command, stopping the penis measurement device (100) from measuring the penis (300).

12. The penis measurement method as claimed in claim 11, wherein after stopping measuring the penis (300), further comprising the following steps:
uploading the erection counter and the flap counter accumulated by the user device (200) to the cloud server, and
displaying the erection counter, the flap counter, and the activity time on the display of the user device (200).

13. The penis measurement method as claimed in claim 11, further comprising the following steps:
recording a total time as the activity time last updated by the user device (200), and displaying the total time;
obtaining a highest value of the hardness value across all times, setting the highest value as a highest hardness value, and displaying the highest hardness value.

14. The penis measurement method as claimed in claim 10, wherein before recording the data receiving time, comprising the following steps:
wirelessly connecting to the communication unit (30) of the penis measurement device (100), and selecting a working mode of the penis measurement device (100) from the plurality of mode options;
calibrating zero for a sensor chip (20) of the penis measurement device (100) with a return-to-zero procedure, and when the return-to-zero procedure is finished, starting to accept receiving the hardness value and the gyroscope value outputted from the penis measurement device (100).
